# EUROPEAN PATENT SPECIFICATION

(11) **EP 0 612 760 B1**
(45) Date of publication and mention of the grant of the patent: **02.01.1997**
(21) Application number: 94301098.3
(22) Date of filing: 16.02.1994
(51) Int. Cl.: C07J 51/00, A61K 31/565, C07J 41/00

(54) **Method for purification of estramustin phosphate or salt thereof**
Verfahren zur Reinigung von Estramustin Phosfat oder Salze davon
Procédé de purification du phosphate d'estramustin ou ses sels

(30) Priority: 18.02.1993 JP 51288/93
(43) Date of publication of application: 31.08.1994
(73) Proprietor: PERMACHEM ASIA, LTD., Chuo-ku Tokyo (JP)
(72) Inventor: Ohwaki, Keiji, Ogasa-gun, Shizuoka-ken (JP); Norose, Satoru, Kakegawa-shi, Shizuoka-ken (JP); Niino, Hideki, Yokohama-shi, Kanagawa-ken (JP)
(74) Representative: Woods, Geoffrey Corlett

(56) References cited:
- EP-A- 0 138 153
- BE-A- 646 319
- FR-A- 2 343 752

## Description

This invention relates to a method for purification of crude estramustin phosphate or a salt thereof. This estramustin phosphate is a common name of estradiol-3-N-bis(β-chloroethyl)-carbamate-17β-dihydrogen phosphate represented by the following formula, and is known to be chemotherapeutically extremely useful as an antitumor agent.

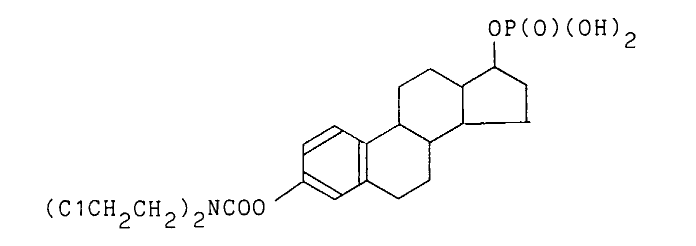

As described in U.K. Patent No. 1,016,959, estramustin phosphate is prepared by reacting estradiol-17-dihydrogen phosphate with N-chloroformyl-bis(β-chloroethyl)amine in the presence of a tertiary amine. However, when the desired compound is isolated and purified from the tertiary amine-containing reaction mixture formed by the reaction, it is known that by recrystallization using methanol, ethanol, acetone or the like as described in the above patent, it is extremely hard to avoid mixing of the tertiary amine, particularly pyridine into the product.

Thus, as a method for removing from purified estramustin starting the above reaction mixture pyridine which is mixing thereinto, a method is proposed in GB 1,523,035 which comprises forming, first, a molecule-like complex is formed from an alkanol having at least three carbon atoms and estramustin phosphate; dissolving the resultant complex in a solvent other than the alcohol used for the complex formation, for example methanol, ethanol or acetonitrile; and recovering the purified desired compound from the solution. Although it is possible to obtain purified estramustin phosphate not containing pyridine or the like according to this proposed method, there is a tendency that the yield lowers due to the interposition of the molecule-like complex formation step. The object of this invention lies in providing a method for purification of estramustin phosphate which is more efficient than the usual methods and can give estramustin phosphate highly pure enough to be usable as a medicinal bulk.

The present inventors found that a purified product derived from a precipitate obtained by simply dissolving crude estramustin phosphate in a fatty acid ester without forming a molecule-like complex with an alkanol, and adding both methanol or ethanol and water in an acidic condition does not contain not only the tertiary amine such as pyridine, but the starting materials nor by-products mixing into the above reaction mixture.

Thus, the above object can be attained by this invention, namely by a method for purification of crude estramustin phosphate which comprises
(A) a step to dissolve the crude estramustin phosphate or a salt thereof in a fatty acid ester,
(B) a step to add both methanol or ethanol or a mixed solvent thereof and water to the resultant solution under an acidic condition to deposit purified estramustin phosphate, and
(C) a step to recover the deposited purified estramustin phosphate, whereby steps (A) and (B) are carried out at a temperature of 60°C or less.

The crude estramustin phosphate according to this invention means estramustin phosphate (hereafter, sometimes abbreviated as "EMP") not having a purity enough as an medicinal raw material, but there are generally included reaction mixtures containing a tertiary amine, particularly pyridine, the starting materials and by-products as described in the aforesaid U.K. Patent No. 1,016,959; and preparatorily purified products wherein pyridine or the like is not sufficiently removed. When EMP exists in the form of a salt, it is preferable to convert it in advance into a free form.

As the fatty acid ester, any one can be used so long as it can dissolved EMP, but there can be mentioned as preferred one an ester derived from a carboxylic acid having up to 5 carbon atoms and an alcohol having up to 4 carbon atoms. Specifically, there can be mentioned a methyl, ethyl, propyl or butyl ester of formic acid, acetic acid, acetoacetic acid, propionic acid or butyric acid or the like.

As an acid for making the solution acidic in the step B according to this invention, there can be mentioned a mineral acid such as hydrochloric acid, sulfuric acid or nitric acid as a preferred one. The use amount of these acids varies depending on the kind of crude EMP and is not limited, but an amount enough to make the solution weakly acidic, i.e., pH value of 6.5 to 6.8, is usually preferable.

The solvent system comprising both methanol or ethanol or a mixed solvent thereof and water to be added to the resultant solution is composed so that the ratio of the former organic solvent to water becomes generally 5 : 95 - 95 : 5, preferably 20 : 80 - 80 : 20, particularly preferably almost the same in volume. As for the addition amount of such solvent system to the solution, the optimum amount varies depending on the pH of the solution, the concentration of EMP, treating temperature, etc., but one skilled in the art will be able to easily determine an optimum amount by carrying out a little experiment taking the deposition rate of EMP from the solution, etc. into account. Each of the above steps is carried out at 60°C or less, conveniently at ambient temperature, but the EPM deposition step can be carried out at a further low temperature.

The thus deposited crystals are recovered by a conventional manner, for example by filtration and dried, and thereby purified estramustin phosphate is obtained in a high yield.

This invention is more specifically described below according to examples.

### Example 1

54.6 g of phosphate oxychloride was dissolved in 280 ml of dry pyridine, the solution was cooled, and a solution obtained by dissolving 33 g of estradiol-3-N-bis(β-chloroethyl)carbamate in 70 ml of dry pyridine was added dropwise to carry out reaction.

The reaction solution was poured in ice water, 1300 ml of 3.5 N hydrochloric acid was added, the mixture was allowed to stand, and the deposited crystals were taken by filtration. The resultant crude estramustin phosphate contained 10% pyridine as an impurity.

These crude estramustin phosphate crystals were dissolved in 480 ml of acetic acid ethyl ester, the solution was made to be acidic with diluted hydrochloric acid, 400 ml of methanol and 400 ml of water were added successively, the mixture was stirred and allowed to stand overnight, and the deposited crystals were taken by filtration and dried to obtain 33.5 g (yield 86%) of purified estramustin phosphate not containing impurities. Melting point 175°C; Angle of rotation [α]D²⁰+31.0°; Purity by liquid chromatography (internal standard method) was 99.3%.

### Example 2

50 g of crude estramustin phosphate disodium salt was dissolved in 500 ml of water, 450 ml of acetic acid ethyl ester was added, the mixture was made to be acidic under stirring, and thereby the suspension gradually became clear and became two layers. The ethyl acetate layer was separated, 600 ml of methanol and 500 ml of water were added successively thereto, and the mixture was treated in the same manner as in Example 1 to obtain 32.7 g of purified estramustin phosphate. Purity by liquid chromatography was 99.4%.

### Example 3

The same operations as in Example 1 were carried out except that acetic acid ethyl ester was changed to acetoacetic acid methyl ester to obtain purified estramustin phosphate.

### Example 4

The same operations as in Example 2 were carried out except that acetic acid ethyl ester and methanol were changed to acetic acid butyl ester and ethanol, respectively to obtain purified estramustin phosphate.

## Claims

1. A method for purification of crude estramustin phosphate which comprises
(A) dissolving the crude estramustin phosphate or a salt thereof in a fatty acid ester,
(B) adding both methanol or ethanol or a mixture thereof and water to the resultant solution under acidic conditions to deposit purified estramustin phosphate, and
(C) recovering the deposited purified estramustin phosphate, whereby steps (A) and (B) are carried out at a temperature of 60°C or less.

2. The method of claim 1 wherein the starting raw material is a crude salt of estramustin phosphate, and the said salt is first converted into free, crude estramustin phosphate prior to step (A).

3. The method of claim 1 or 2 wherein the crude estramustin phosphate or salt thereof contains a tertiary amine as an impurity.

4. The method of claim 3 wherein the said tertiary amine is pyridine.

5. The method of any one of claims 1 to 4 wherein the fatty acid ester is derived from a carboxylic acid having up to 5 carbon atoms and an alcohol having up to 4 carbon atoms.

6. The method of any one of claims 1 to 5 wherein the acidic conditions of step (B) are adjusted with hydrochloric acid.

## Patentansprüche

1. Verfahren zur Reinigung eines Roh-Estramustinphosphats, welches umfaßt
(A) Lösen des Roh-Estramustinphosphats oder eines Salzes davon in einem Fettsäureester,
(B) Zugeben sowohl von Methanol oder Ethanol oder eines Gemischs davon als auch von Wasser zur resultierenden Lösung unter sauren Bedingungen zur Ausfällung von gereinigtem Estramustinphosphat, und
(C) Rückgewinnen des ausgefällten gereinigten Estramustinphosphats,
wobei die Schritte (A) und (B) bei einer Temperatur von 60°C oder weniger durchgeführt werden.

2. Verfahren nach Anspruch 1, worin der Ausgangs-Rohstoff ein Rohsalz von Estramustinphosphat ist und dieses Salz zunächst in freies Roh-Estramustinphosphat vor Schritt (A) umgewandelt wird.

3. Verfahren nach Anspruch 1 oder 2, worin das Roh-Estramustinphosphat oder dessen Salz ein tertiäres Amin als Unreinheit enthält.

4. Verfahren nach Anspruch 3, worin dieses tertiäre Amin Pyridin ist.

5. Verfahren nach einem der Ansprüche 1 bis 4, worin der Fettsäureester aus einer Carboxylsäure mit bis zu 5 Kohlenstoffatomen und einem Alkohol mit bis zu 4 Kohlenstoffatomen stammt.

6. Verfahren nach einem der Ansprüche 1 bis 5, worin die sauren Bedingungen des Schritts (B) mit Salzsäure eingestellt werden.

## Revendications

1. Procédé de purification du phosphate d'estramustine brut, qui comprend :
(A) la dissolution du phosphate d'estramustine brut ou de l'un de ses sels dans un ester d'acide gras,
(B) l'addition à la fois de méthanol ou d'éthanol ou d'un mélange des deux et d'eau à la solution résultante, dans des conditions acides, pour faire précipiter le phosphate d'estramustine purifié, et
(C) la récupération du phosphate d'estramustine purifié précipité,
les étapes (A) et (B) étant réalisées à une température inférieure ou égale à 60°C.

2. Procédé selon la revendication 1, dans lequel la matière première de départ est un sel brut de phosphate d'estramustine et ledit sel est d'abord transformé en phosphate d'estramustine brut, libre, avant l'étape (A).

3. Procédé selon la revendication 1 ou 2, dans lequel le phosphate d'estramustine brut ou son sel contient une amine tertiaire comme impureté.

4. Procédé selon la revendication 3, dans lequel l'amine tertiaire est la pyridine.

5. Procédé selon l'une quelconque des revendications 1 à 4, dans lequel l'ester d'acide gras est issu d'un acide carboxylique ayant jusqu'à 5 atomes de carbone et d'un alcool ayant jusqu'à 4 atomes de carbone.

6. Procédé selon l'une quelconque des revendications 1 à 5, dans lequel les conditions acides de l'étape (B) sont réglées par addition d'acide chlorhydrique.
